# EUROPÄISCHE PATENTANMELDUNG

(11) **EP 4 086 667 A1**
(43) Veröffentlichungstag der Anmeldung: **09.11.2022**
(21) Anmeldenummer: 22166624.1
(22) Anmeldetag: 05.04.2022
(51) Int. Cl.: G01T 7/00, A61N 5/10

(54) **VERFAHREN SOWIE VORRICHTUNG ZUR VALIDIERUNG DER ACHSLINEARITÄT UND/ODER DER POSITIONIERGENAUIGKEIT EINES VERSTELLMECHANISMUS FÜR EINEN STRAHLENDETEKTOR**

(30) Priorität: 06.05.2021 DE 102021111815
(71) Anmelder: LAP GmbH Laser Applikationen, 21337 Lüneburg (DE)
(72) Erfinder: Brunk, Markus, 21398 Neetze (DE); Bojara, Daniel, verstorben (DE)
(74) Vertreter: Hauck Patentanwaltspartnerschaft mbB

(57) **Zusammenfassung**

Verfahren und Vorrichtung zur Validierung der Achslinearität und/oder der Positioniergenauigkeit eines Verstellmechanismus für einen Strahlendetektor zum Detektieren von von einem Bestrahlungsgerät ausgesendeter hochenergetischer Strahlung, mit den Schritten: Verstellen eines innerhalb eines Behälters zur Aufnahme einer Flüssigkeit angeordneten, taktilen Sensorelements und/oder eines innerhalb des Behälters angeordneten Normelements über den Verstellmechanismus entlang zumindest einer Raumachse zum taktilen Erfassen des Normelements mittels des taktilen Sensorelements..

## Beschreibung

Die Erfindung betrifft ein Verfahren sowie eine Vorrichtung zur Validierung der Achslinearität und/oder der Positioniergenauigkeit eines Verstellmechanismus für einen Strahlendetektor, der zum Detektieren von von einem Bestrahlungsgerät ausgesendeter hochenergetischer Strahlung ausgebildet ist.

Im medizinischen Bereich werden Bestrahlungsgeräte zur Behandlung von bösartigem Gewebe eingesetzt. So wird beispielsweise mittels eines Linearbeschleunigers (LINAC = *linear particle accelerator*) erzeugte, hochbeschleunigte Teilchenstrahlung bzw. elektromagnetische Strahlung auf das zu behandelnde Gewebe gerichtet. Eine solche Strahlenbehandlung muss sowohl hinsichtlich des Auftreffortes der Strahlung, als auch hinsichtlich der Intensität der Strahlung am Auftreffort sehr präzise durchgeführt werden, um eine Beschädigung gesunden Gewebes zu vermeiden. Zur Planung derartiger Behandlungen werden Computersysteme verwendet, die auf möglichst exakte Daten angewiesen sind, insbesondere in Bezug auf den Auftreffort sowie die Intensität der Strahlung an dem Auftreffort, also am Ort der beabsichtigten Wechselwirkung zwischen Strahlung und dem zu behandelnden Gewebe.

Vor der Bestrahlung wird daher in der Regel eine Messung der von der Strahlungsquelle erzeugten Strahlung insbesondere hinsichtlich ihrer Intensität sowie auch hinsichtlich ihres Auftrefforts durchgeführt. Dabei wird üblicherweise die Strahlungsmessung unter Verwendung eines Bestrahlungsziels durchgeführt, welches eine mit dem menschlichen Körper vergleichbare Dichte aufweist. So wird typischerweise ein mit Wasser gefüllter Behälter, auch Wasserphantom genannt, als Bestrahlungsziel verwendet, wobei innerhalb des Wasserphantoms ein Detektor zum Erfassen der Strahlung angeordnet ist. Der Detektor ist dabei üblicherweise innerhalb des Wasserphantoms in allen drei Raumrichtungen X, Y, Z beweglich angeordnet, um die Intensität der Strahlung an verschiedenen Orten innerhalb des Wasserphantoms messen zu können. Zum Bewegen des Detektors entlang der drei zueinander orthogonalen Raumrichtungen wird ein Verstellmechanismus verwendet, der einen oder mehrere auf Schienen verfahrbare Schlitten umfassen kann, wobei an einer der Schienen der Detektor angeordnet ist. Es sind zumeist drei Schlitten vorgesehen, um den Detektor entlang der drei zueinander orthogonalen Raumrichtungen verfahren zu können.

Damit der Strahlendetektor aussagekräftige Daten liefert, ist insbesondere sicherzustellen, dass der Verstellmechanismus den Detektor in möglichst exakter Weise in Übereinstimmung mit einer Vorgabe verstellt. So kann beispielsweise eine Steuereinheit eine Bewegung um 10 mm entlang der X-Achse vorgeben und den Verstellmechanismus zur Bewegung des Strahlendetektors entsprechend ansteuern. Bei der Bewegung des Strahlendetektors und insbesondere des ihn tragenden Schlittens entlang der jeweiligen Bewegungsachse kann es jedoch zu unerwünschten Abweichungen kommen. Daher ist eine regelmäßige Überprüfung der tatsächlichen durch den Verstellmechanismus und damit den Strahlendetektor eingenommenen Achspositionund/oder der Achslinearität der Bewegung desselben von wesentlicher Bedeutung.

Bekannte Systeme zur Validierung der Achslinearität sehen ein außerhalb des Wasserphantoms angeordnetes interferometrisches Vermessungssystem vor, beispielsweise umfassend einen Vermessungslaser, der auf den Strahlendetektor bzw. den ihn tragenden Schlitten gerichtet ist. Derartige Systeme haben jedoch mehrere Nachteile. So kann mit einem solchen externen System die Achslinearität des Strahlendetektors nicht unter Realbedingungen ermittelt werden. Denn zum einen kann das Wasserphantom in seinem mit Wasser gefüllten Zustand die Messung beeinträchtigen. Zum anderen werden die erwähnten Strahlungsquellen zur Behandlung von bösartigem Gewebe häufig in Kombination mit einem Magnetresonanztomographen (MRT) und damit in einem starken Magnetfeld eingesetzt. Idealerweise wird das Wasserphantom dabei auf die Patientenliege verbracht, auf welcher später dann der für die Behandlung vorgesehene Patient liegen soll. Eine solche Positionierung des Wasserphantoms innerhalb des MRT verhindert jedoch schon aus Platzgründen eine Messung mit einem solchen externen Vermessungssystem. Zudem beeinträchtigt das starke Magnetfeld das externe Vermessungssystem. Die Bauteile des externen Vermessungssystems MR-tauglich auszulegen ist aufwändig und kostenintensiv.

Ausgehend von diesem Stand der Technik liegt der Erfindung die Aufgabe zugrunde, ein Verfahren sowie eine Vorrichtung zur Validierung der Achslinearität eines Strahlendetektors bereitzustellen, die eine besonders zuverlässige Validierung ermöglichen, insbesondere auch innerhalb eines externen starken Magnetfelds sowie unter Wasser.

Die Erfindung löst die Aufgabe durch ein Verfahren gemäß Anspruch 1 sowie durch eine Vorrichtung gemäß Anspruch 7. Vorteilhafte Ausgestaltungen sind Gegenstand der abhängigen Ansprüche, der Beschreibung sowie der Figuren.

Das erfindungsgemäße Verfahren der eingangs genannten Art umfasst die Schritte: Verstellen eines innerhalb eines Behälters zur Aufnahme einer Flüssigkeit angeordneten, taktilen Sensorelements über einen Verstellmechanismus entlang zumindest einer Raumachse zum taktilen Erfassen eines innerhalb des Behälters angeordneten Normelements. Alternativ oder zusätzlich kann das Verfahren den Schritt umfassen: Verstellen eines innerhalb des Behälters zur Aufnahme einer Flüssigkeit angeordneten Normelements über den Verstellmechanismus entlang zumindest einer Raumachse zum taktilen Erfassen des Normelements durch das taktile Sensorelement.

Die erfindungsgemäße Vorrichtung der eingangs genannten Art umfasst einen Behälter zur Aufnahme einer Flüssigkeit, den Verstellmechanismus und ein innerhalb des Behälters angeordnetes, taktiles Sensorelement, wobei das Sensorelement zum taktilen Erfassen eines innerhalb des Behälters angeordneten Normelements ausgebildet ist. Das Sensorelement und/oder das Normelement sind über den Verstellmechanismus entlang zumindest einer Raumachse relativ zueinander verstellbar. So kann das Sensorelement in Kontakt mit dem Normelement gebracht werden.

Bei dem erfindungsgemäßen Verfahren und der erfindungsgemäßen Vorrichtung können also Sensorelement und Normelement relativ zueinander bewegt werden. Dabei kann das Sensorelement über den Verstellmechanismus beweglich sein und das Normelement räumlich fixiert, insbesondere in Bezug auf den Behälter räumlich fixiert. Auch kann das Normelement über den Verstellmechanismus beweglich sein und das Sensorelement räumlich fixiert, insbesondere in Bezug auf den Behälter räumlich fixiert. Auch können grundsätzlich sowohl das Sensorelement und das Normelement beweglich sein, zumindest eines der beiden über den Verstellmechanismus.

Die erfindungsgemäße Vorrichtung ist zur Ausführung des erfindungsgemäßen Verfahrens geeignet. Insbesondere kann die Vorrichtung eine Steuereinheit umfassen, die zur Durchführung des erfindungsgemäßen Verfahrens dient. Das Verfahren und die Vorrichtung werden nachfolgend gemeinsam erläutert. Zum Verfahren gemachte Erläuterungen gelten folglich entsprechend auch für die Vorrichtung und andersherum.

Die erfindungsgemäße Vorrichtung umfasst einen Behälter zur Aufnahme einer Flüssigkeit, insbesondere zur Aufnahme von Wasser, sowie einen Verstellmechanismus. Der Behälter mitsamt dem Verstellmechanismus wird im Weiteren auch als Wasserphantom bezeichnet, wobei jedoch anstatt von Wasser auch grundsätzlich eine andere Flüssigkeit verwendet werden kann. Zudem umfasst die Vorrichtung ein taktiles Sensorelement, das über einen Verstellmechanismus entlang zumindest einer Raumachse, bevorzugt jedoch entlang von zwei oder drei zueinander orthogonalen Raumachsen verstellbar ist. Alternativ kann auch das Normelement entsprechend verstellbar sein, wie oben erläutert. Aus Gründen der Vereinfachung wird im Folgenden zumeist von dem Verstellen oder Bewegen des Sensorelements mittels des Verstellmechanismus gesprochen, wobei dem Fachmann klar ist, dass alternativ auch das Verstellen oder Bewegen des Normelements relativ zu dem Sensorelement gemeint ist. Hierfür kann der Verstellmechanismus einen oder mehrere Schlitten aufweisen, die relativ zueinander beweglich sind. Dadurch ist das Sensorelement bzw. das Normelement innerhalb des Behälters relativ zu dem Behälter über den Verstellmechanismus beweglich angeordnet. Auch der Verstellmechanismus selbst, insbesondere dessen Schlitten, sind bevorzugt innerhalb des Behälters angeordnet.

Die erfindungsgemäße Vorrichtung mit ihrem Wasserphantom dient in eingangs erläuterter Weise zur Ersteinrichtung bzw. Qualitätssicherung von Linearbeschleunigern, insbesondere von sogenannten Magnetresonanz-Linearbeschleunigern (MR-LINACs). So muss auch in MR-LINAC Bestrahlungsräumen im Magnetischen Feld im Rahmen der Kommissionierung die Achspositioniergenauigkeit von Wasserphantomen nachgewiesen werden. MR-LINACs vereinen die Bestrahlungseinheit, also den Linearbeschleuniger, und das MRT in einem Gerät, wie eingangs bereits angesprochen. Es kann so das Bestrahlungsobjekt, beispielsweise ein Tumor, und die angrenzenden gesunden Normalgewebe mittels MR sichtbar gemacht und der Behandlungsstrahl anschließend exakt auf den Tumor gerichtet werden. Umliegendes Gewebe kann somit geschont werden. Beispielsweise kann das taktile Sensorelement anstelle des Strahlendetektors an dem Verstellmechanismus angeordnet sein, wie später noch erläutert wird. Der Verstellmechanismus des taktilen Sensorelements ist insbesondere derselbe, der bei der üblichen Verwendung des Wasserphantoms den Strahlendetektor trägt und verstellt. Der Verstellmechanismus kann beispielsweise ein Adapter- oder Aufnahmeelement aufweisen, welches das taktile Sensorelement trägt.

Mittels des über den Verstellmechanismus verstellbaren taktilen Sensorelements bzw. Normelements kann die Achslinearität sowie die Positioniergenauigkeit des Verstellmechanismus des Strahlendetektors, und damit die Achslinearität sowie die Positioniergenauigkeit des Strahlendetektors selbst, validiert werden. Validierung der Positioniergenauigkeit meint dabei insbesondere ein Überprüfen, ob eine vorgegebene Position im Raum auch exakt erreicht wird. Validierung der Achslinearität meint insbesondere ein Überprüfen, ob eine angeforderte Bewegung in der gewünschten Weise umgesetzt wird, ob also beispielsweise in der einleitend bereits angedeuteten Weise eine Bewegung des Strahlendetektors bzw. des ihn tragenden Teils des Verstellmechanismus zwischen zwei Punkten entsprechend einer vorgegebenen Distanz erfolgt. Insbesondere kann an mehreren Positionen entlang einer Bewegungsachse des Sensorelements bzw. des Verstellmechanismus eine Positionsbestimmung durchgeführt werden. Es können in diesem Sinne eine oder mehrere Achspositionen validiert werden. Auf deren Grundlage kann die Validierung der Achslinearität erfolgen. Es kann eine Steuereinheit vorgesehen sein zur Ansteuerung des Verstellmechanismus zur Bewegung des Sensorelements. Diese kann dazu ausgebildet sein, eine Bewegung des Verstellmechanismus und damit des Sensorelements vorzugeben sowie insbesondere die Einhaltung dieser Bewegung zu überprüfen und gegebenenfalls nachzuregeln. Es kann also auch eine Kalibrierung des Verstellmechanismus vorgesehen sein.

Die Erfindung zeichnet sich gegenüber dem Stand der Technik dadurch aus, dass zum einen ein taktiles Sensorelement vorgesehen ist, eine Positionsbestimmung also taktil erfolgt, sowie zum anderen dadurch, dass dieses taktile Sensorelement innerhalb des Wasserphantoms angeordnet ist. Mittels des taktilen Sensorelements kann ein ebenfalls innerhalb des Wasserphantoms angeordnetes Normelement taktil erfasst werden. Das Sensorelement kann beispielsweise einen Sensorkopf umfassen, der in Kontakt mit dem Normelement gebracht wird zum taktilen Erfassen des Normelements. Als Normelement ist ein Element zu verstehen, dessen Vermessung oder Erfassung durch das Sensorelement einen Rückschluss auf die Position des Verstellmechanismus und/oder auf eine durch den Verstellmechanismus zurückgelegte Distanz erlaubt. Als Normelement ist insbesondere ein Element mit definierten Abmessungen zu verstehen. Dabei muss es sich nicht um ein einer Norm, wie beispielsweise einer DIN-Norm, zugrundeliegendes Normelement handeln. Insbesondere kann das Normelement als eine Maßverkörperung aufgefasst werden. Mittels der Erfindung kann also eine Validierung der Achsposition bzw. der Achslinearität des Sensorelements bzw. des Verstellmechanismus erfolgen. Im Unterschied zum Stand der Technik ist also kein externes Vermessungssystem vorgesehen, wie beispielsweise ein auf den Strahlendetektor im Wasserphantom gerichteter Laser. Stattdessen ist das zur Validierung vorgesehene System innerhalb des Wasserphantoms angeordnet, insbesondere integriert. In anderen Worten liegt der Erfindung der Gedanke zugrunde, dass Wasserphantom, einschließlich dessen Behälter und dessen Verstellmechanismus, zur Validierung zu verwenden, indem über das taktile Sensorelement innerhalb des Behälters ein Normelement erfasst wird.

Es kann somit unter Realbedingungen, nämlich insbesondere innerhalb des wassergefüllten Behälters zum einen sowie innerhalb des durch das MRT erzeugten externen Magnetfelds gearbeitet werden. Die gesamte Vorrichtung kann zur Validierung auf die Patientenliege des MR-LINACs verbracht werden und unter Realbedingungen, also insbesondere bei aktivem Magnetfeld des MRT und ggf. auch bei wassergefülltem Phantom, die Validierung durchführen. Da es sich erfindungsgemäß um ein taktiles Sensorelement handelt, stört das externe Magnetfeld nicht die Validierung. So ist das taktile Erfassen des innerhalb des Behälters angeordneten Normelements durch das taktile Sensorelement auch bei einem starken externen Magnetfeld ohne nennenswerte Beeinträchtigung der Messgenauigkeit möglich. Das erfindungsgemäße Verfahren bzw. die erfindungsgemäße Vorrichtung erlauben somit unter realen Bedingungen eine zuverlässige Validierung der jeweils angesteuerten Achsposition und der Achslinearität des Verstellmechanismus und damit des Strahlendetektors. Die Komponenten des erfindungsgemäßen Systems, insbesondere das taktile Sensorelement und das Normelement, können in kostengünstiger Weise MR-tauglich ausgelegt werden, also widerstandsfähig gegenüber dem externen Magnetfeld des MRT. So können mechanische Bauteile wie die Normelemente beispielsweise aus Keramik oder Aluminium bestehen. Ein Sensorkopf des Sensorelements kann insbesondere aus nicht-ferromagnetischem Metall bestehen oder als einfaches elektrisches Bauteil ausgebildet sein, insbesondere als Kontaktschalter. Nach der Durchführung dieser Validierung kann über den Strahlendetektor in an sich bekannterweise überprüft werden, ob der Behandlungsstrahl des Linearbeschleunigers am gewünschten Ort mit der gewünschten Intensität auftrifft.

Nach einer Ausgestaltung ist das taktile Sensorelement entlang zweier oder dreier zueinander orthogonaler Raumachsen zum taktilen Erfassen des Normelements verstellbar ausgebildet. Entsprechend kann das Verfahren den Schritt umfassen: Verstellen des taktilen Sensorelements entlang zweier oder dreier zueinander orthogonaler Raumachsen zum taktilen Erfassen des Normelements. Der Verstellmechanismus kann entsprechend ausgebildet sein. So kann der Verstellmechanismus beispielsweise zwei oder drei relativ zueinander bewegliche Schlitten umfassen, welche die Bewegung des am Verstellmechanismus getragenen Sensorelements entlang der zwei oder drei zueinander orthogonalen Raumachsen ermöglichen. Das Verstellen entlang der zueinander orthogonalen Achsen meint dabei ein Verstellen derart, dass das Sensorelement von einer ersten Position X₁, Y₁, Z₁ zu einer zweiten Position X₂, Y₃, Z₃ bewegt wird. Dies kann in drei zueinander senkrechten Bewegungsschritten entlang der jeweiligen Achsen erfolgen oder auch in einer gemeinsamen Bewegung, beispielsweise entlang einer Diagonalen. Auch kann dies, in sphärischen Koordinaten, erreicht werden durch Bewegen entlang einer Achse und Änderung zweier Winkel.

Nach einer Ausgestaltung ist das Normelement als Prüfplatte mit Vertiefungen und/oder Erhebungen ausgebildet, wobei das Sensorelement zum taktilen Erfassen der Prüfplatte entlang der Vertiefungen und/oder Erhebungen gefahren wird. Auch kann grundsätzlich die Prüfplatte mittels des Verstellmechanismus verfahrbar sein, also derart entlang des Sensorelements bewegt werden, dass dessen Sensorkopf die Vertiefungen und/oder Erhebungen der Prüfplatte erkennt. Die Prüfplatte kann innerhalb des Wasserphantoms derart angeordnet sein, dass das taktile Sensorelement entlang der Prüfplatte verfahren werden kann. Die Prüfplatte kann beispielsweise Vertiefungen in Form von Nuten aufweisen. Ein Sensorkopf des Sensorelements kann beispielsweise in eine solche Vertiefung hineinverfahren und anschließend entlang einer Längserstreckung der Vertiefung bewegt werden. Der Sensorkopf kann als Tastkopf insbesondere aus nicht-ferromagnetischem Metall bestehen. Es können beispielsweise kreisförmige Vertiefungen bzw. Erhebungen, beispielsweise kreisringförmige Nuten, vorgesehen sein oder auch rechteckförmige. Es kann also somit insbesondere zumindest entlang zweier zueinander orthogonaler Raumachsen das Sensorelement entlang der Vertiefungen bzw. Erhebungen der Prüfplatte verfahren werden. Beispielsweise kann die Prüfplatte in der X-Z-Ebene angeordnet sein, sodass das taktile Sensorelement entlang der ebenfalls in dieser Ebene angeordneten Vertiefungen bzw. Erhebungen verfahren werden kann. Somit kann zumindest bezogen auf die X- und Z-Achse die Achsposition bzw. Achslinearität des Verstellmechanismus und damit des Strahlendetektors validiert werden. Auch kann die Prüfplatte, beispielsweise in einem nachfolgenden Verfahrensschritt, anderweitig innerhalb des Behälters positioniert und erneut durch das Sensorelement abgefahren werden. So kann beispielsweise die Prüfplatte entlang der Y-Z-Ebene ausgerichtet werden. Ein Abfahren der Vertiefungen bzw. Erhebungen der Prüfplatte durch das Sensorelement erlaubt dann auch eine Validierung der Achsposition bzw. Achslinearität entlang der Y-Achse.

Nach einer Ausgestaltung umfasst das Normelement mindestens ein Endmaß, wobei das Sensorelement zum taktilen Erfassen des Endmaßes in Kontakt mit dem Endmaß verstellt wird. Auch kann grundsätzlich das Endmaß verstellbar sein, also das Endmaß an das Sensorelement herangefahren werden. Der Sensorkopf des Sensorelements kann in diesem Fall als einfaches elektrisches Bauteil ausgebildet sein, insbesondere als Kontaktschalter. Dabei kann das taktile Sensorelement insbesondere über den Verstellmechanismus entlang einer der drei Raumachsen verstellt werden, also von einer Ausgangsposition beabstandet zu dem Endmaß hin zu einer Endposition in Kontakt mit dem Endmaß. Das Endmaß weist als Normelement eine exakt definierte Länge auf, wie erwähnt, was eine Validierung der Achsposition bzw. Achslinearität ermöglicht, insbesondere wenn in mehreren Schritten mehrere Endmaße derart taktil erfasst werden. Die Endmaße können beispielsweise aus Keramik bestehen.

So ist nach einer Ausgestaltung vorgesehen, dass das Normelement mehrere Endmaße umfasst, die in mehreren Schritten nacheinander durch das Sensorelement taktil erfasst werden. Auch können die Endmaße stattdessen die Endmaße verfahren werden. Die Endmaße können dieselben oder unterschiedliche Längen aufweisen. Die Endmaße können beispielsweise in einer Endmaßführung aufgenommen sein, die entlang einer der drei Raumachsen ausgerichtet ist. Zur Validierung der Achsposition bzw. Achslinearität kann beispielsweise zunächst ein erstes Endmaß einer ersten Länge vorgelegt und durch das Sensorelement erfasst, also durch das Sensorelement angefahren werden. Anschließend kann das erste Endmaß ersetzt werden durch ein zweites Endmaß einer größeren, zweiten Länge, wobei das zweite Endmaß wiederum durch das Sensorelement erfasst, also angefahren wird. Auch kann das zweite Endmaß an das erste Endmaß angelegt und anschließend erfasst werden. Das Sensorelement fährt somit in aufeinanderfolgenden Schritten unterschiedlich lange Wege ab. Das Sensorelement kann in diesem Fall beispielsweise einen Kontaktschalter als Sensorkopf umfassen, der bei Kontakt mit dem jeweiligen Endmaß auslöst. Im Rahmen der Validierung der Achsposition bzw. Achslinearität wird dann jeweils die Auslöseposition, bei der das Sensorelement aufgrund der taktilen Erfassung des jeweiligen Endmaßes auslöst, ermittelt. Die Auslöseposition kann automatisch durch die Steuereinheit erfasst werden, oder kann grundsätzlich auch manuell notiert werden. Mithilfe der normierten Endmaße kann somit in einfacher Weise und ebenfalls innerhalb des externen Magnetfelds und innerhalb des Wasserphantoms die Validierung erfolgen. Das Verfahren wird später noch im Detail erläutert.

Nach einer diesbezüglichen Ausgestaltung finden einige der Schritte außerhalb und einige der Schritte innerhalb des Magnetfelds eines Magnetresonanztomographen (MRT) statt. Insbesondere können auch alle Schritte innerhalb des Magnetfelds stattfinden. Beispielsweise kann ein erstes Endmaß bei außerhalb des externen Magnetfelds angeordneter Vorrichtung angefahren und ein zweites Endmaß bei innerhalb des externen Magnetfelds des MRT angeordneter Vorrichtung angefahren werden. Die Vorrichtung kann hierfür beispielsweise auf einer beweglichen Patientenliege des MRT bzw. MR-LINAC angeordnet werden, wobei diese einmal in das Magnetfeld und einmal aus dem Magnetfeld herausgefahren wird. Dies kann die Validierung noch verbessern, wie durch die später noch folgende Detailerläuterung deutlich wird.

Nach einer Ausgestaltung ist das taktile Sensorelement an der für den Strahlendetektor vorgesehenen Stelle an dem Verstellmechanismus angeordnet. Insbesondere kann das taktile Sensorelement an dem bereits erwähnten Aufnahmeelement, welches ansonsten den Strahlendetektor trägt, angeordnet sein. Das Aufnahmeelement kann also zur wahlweisen Aufnahme des Strahlendetektors und des Sensorelements ausgebildet sein. Durch Positionierung des taktilen Sensorelements an der für den Strahlendetektor vorgesehenen Stelle an dem Verstellmechanismus kann eine besonders sichere Validierung erreicht werden.

Nach einer Ausgestaltung umfasst die Vorrichtung eine Steuereinheit zur Durchführung des Verfahrens, wie bereits angesprochen. Das Verfahren kann somit teilweise, bevorzugt vollständig, automatisch ausgeführt werden. Insbesondere kann über die Steuereinheit automatisch das Sensorelement bzw. das Normelement verstellt werden, also der Verstellmechanismus entsprechend angesteuert werden. So kann beispielsweise bei einer Prüfplatte als Normelement das Sensorelement entlang der Vertiefungen bzw. Erhebungen automatisch über die Steuereinheit bewegt werden. Auch können bei einer Ausbildung der Normelemente als Endmaße diese automatisch durch das Sensorelement angefahren werden. Das erfindungsgemäße Verfahren kann somit zumindest teilweise automatisiert werden.

Ausgestaltungen der Erfindung werden im Folgenden anhand von Figuren erläutert. Es zeigen:
- Fig. 1: eine erfindungsgemäße Vorrichtung gemäß einer ersten Ausgestaltung,
- Fig. 2a, b: das als Prüfplatte ausgebildete Normelement aus Figur 1,
- Fig. 3: eine erfindungsgemäße Vorrichtung gemäß einer weiteren Ausgestaltung mit Endmaßen als Normelementen zur Validierung der Achsposition bzw. Achslinearität entlang der Y-Achse,
- Fig. 4a-c: mehrere Endmaße als Normelemente gemäß der Vorrichtung aus Figur 3,
- Fig. 5: eine erfindungsgemäße Vorrichtung ebenfalls mit Endmaßen als Normelementen zur Validierung der Achsposition bzw. Achslinearität entlang der X-Achse, und
- Fig. 6: eine erfindungsgemäße Vorrichtung ebenfalls mit Endmaßen als Normelementen zur Validierung der Achsposition bzw. Achslinearität entlang der Z-Achse.

Soweit nichts anderes angegeben ist, bezeichnen im Folgenden gleiche Bezugszeichen gleiche Gegenstände.

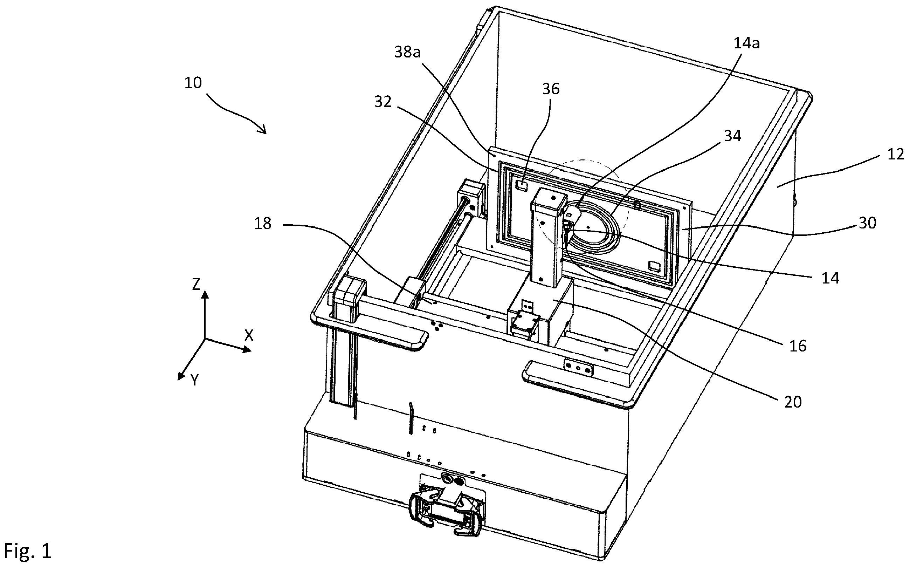

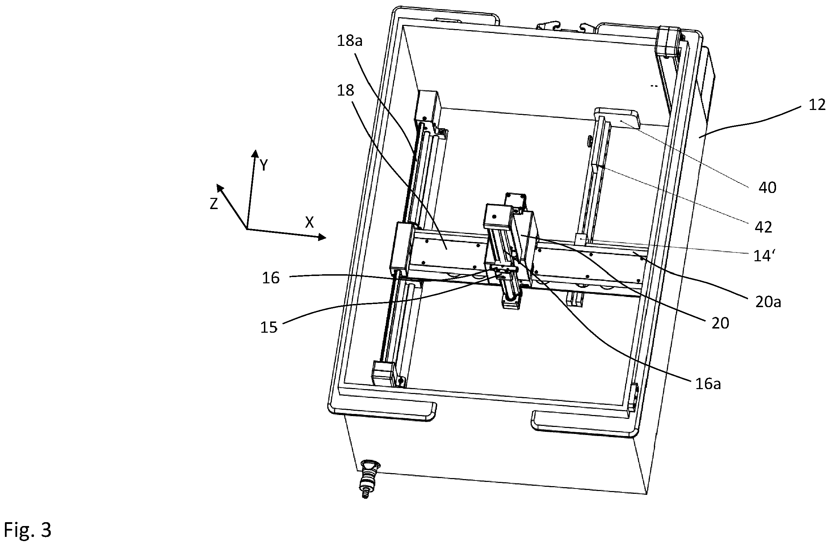

In Figur 1 ist eine erste Ausgestaltung der erfindungsgemäßen Vorrichtung ersichtlich. Die Vorrichtung 10 zeigt ein Wasserphantom umfassend einen Behälter 12, der mit Wasser gefüllt werden kann. Innerhalb des Behälters 12 ist ein taktiles Sensorelement 14 mit einem Sensorkopf 14a angeordnet. Das Sensorelement 14 ist über einen Verstellmechanismus 16, 18, 20 verstellbar, der drei Schlitten umfasst, mit denen das Sensorelement 14 entlang der drei zueinander orthogonalen Raumachsen X, Y und Z verstellt werden kann. So ist das Sensorelement 14 über einen ersten Schlitten 16 entlang der Z-Achse, über einen zweiten Schlitten 18 entlang der Y-Achse und über einen dritten Schlitten 20 entlang der X-Achse verstellbar. Dabei läuft der erste Schlitten 16 auf einer ersten Schiene 16a, der zweite Schlitten 18 auf einer zweiten Schiene 18a und der dritte Schlitten 20 auf einer dritten Schiene 20a (siehe Fig. 3). Die Schienen 16a, 18a, 20a verlaufen allesamt orthogonal zueinander entlang jeweils einer der drei Raumrichtungen. Die drei Schlitten 16, 18, 20 sowie die Schienen 16a, 18a, 20a des Verstellmechanismus sind ebenfalls innerhalb des Behälters 12 angeordnet und können als Teil des Wasserphantoms verstanden werden.

Zudem befindet sich innerhalb des Behälters 12 ein bei dieser Ausgestaltung als Prüfplatte 30 ausgebildetes Normelement. Die Prüfplatte 30 weist eine rechteckige Form auf und verfügt über mehrere darin ausgebildete Vertiefungen, nämlich in der Form von Rechtecken verlaufende Nuten 32, die sich entlang von Randbereichen der Prüfplatte 30 erstrecken, zentral in der Prüfplatte 30 angeordnete kreisringförmige Nuten 34 sowie in einander gegenüberliegenden Eckbereichen der Prüfplatte angeordneten rechteckförmigen Vertiefungen 36. Zudem sind in den Ecken der Prüfplatte 30 Bohrungen 38a und mittig eine Zentralbohrung 38b ausgebildet.

Das Wasserphantom dient im Allgemeinen der Prüfung, ob von einem externen Bestrahlungsgerät ausgesendete hochenergetische Strahlung an einer gewünschten Position in einer gewünschten Intensität ankommt. Bei dem Bestrahlungsgerät handelt es sich insbesondere um einen Linearbeschleuniger, der bevorzugt in Kombination mit einem MRT verwendet wird. Mittels derartiger MR-LINACs kann über das MRT ein zu bestrahlendes Objekt, wie beispielsweise ein Tumor, konkret identifiziert und anschließend mittels des durch den Linearbeschleuniger ausgesendeten Behandlungsstrahls exakt fokussiert werden. In regelmäßigen Abständen und insbesondere zur Planung der Behandlung wird mit einem einen Strahlendetektor umfassenden Wasserphantom geprüft, ob der Behandlungsstrahl auch tatsächlich an der gewünschten Position in der gewünschten Intensität auftrifft. Hierzu kann das Wasserphantom an dem Verstellmechanismus 16, 18, 20 einen Strahlendetektor aufweisen. Für eine aussagekräftige Überprüfung ist es von wesentlicher Bedeutung, dass der Verstellmechanismus den Strahlendetektor in exakt der vorgegebenen Weise verfährt. Hierfür muss die Achsposition bzw. Achslinearität des Strahlendetektors validiert werden.

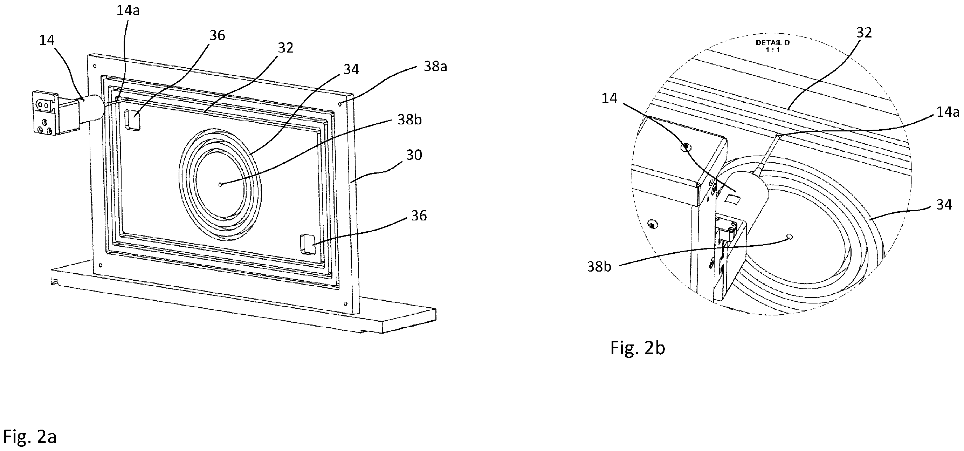

Die erfindungsgemäße Vorrichtung ermöglicht eine solche Validierung. Hierfür ist in der Ausgestaltung nach den Figuren 1 und 2 an der ansonsten für den Strahlendetektor vorgesehenen Stelle des Verstellmechanismus das Sensorelement 14 angeordnet. So kann der Schlitten 16 ein nicht dargestelltes Aufnahmeelement aufweisen, welches üblicherweise den Strahlendetektor trägt, vorliegend jedoch anstelle des Strahlendetektors das Sensorelement 14 aufweist. Durch Abfahren der Prüfplatte 30 mittels des Sensorelements 14 kann die Achsposition bzw. Achslinearität des Verstellmechanismus und damit auch des Strahlendetektors validiert werden. Insbesondere kann überprüft werden, ob der Verstellmechanismus eine gewünschte Bewegungsanforderung in exakt der gewünschten Weise umsetzt. Falls nicht, kann nachjustiert bzw. kalibriert werden.

Bei der Ausgestaltung aus den Figuren 1 und 2 fährt das taktile Sensorelement 14 hierfür mit seinem Sensorkopf 14a entlang der Nuten 32, 34. Zur initialen Ausrichtung des Sensorelements 14 zu der Prüfplatte 30 können zuvor die rechteckförmigen Vertiefungen 36 als Startpunkt angefahren werden. Die Dimensionen des Sensorkopfes 14a und der Nuten 32, 34 sind dabei derart aufeinander abgestimmt, dass eine vorgegebene Toleranz der Achslinearität eingehalten wird. Auch können punktuelle Prüfungen an den in den Ecken der Prüfplatte 30 angeordneten Bohrungen 38a und an eine Zentralbohrung 38b vorgenommen werden. Der Sensorkopf 14a und die Prüfplatte 30 können elektrisch leitend ausgebildet sein, sodass eine Berührung der Prüfplatte 30 durch den Sensorkopf 14a durch eine entsprechende Sensorik festgestellt werden kann. Insbesondere kann ein wasserdichter Tastkopf als Sensorkopf vorgesehen sein, sodass auch bei mit Wasser gefülltem Behälter die Durchführung des Verfahrens möglich ist. Über eine Steuereinheit kann nun das Sensorelement entlang der Nuten 32, 34 verfahren werden und somit die Achsposition bzw. Achslinearität des Verstellmechanismus in der X-Z-Ebene validiert werden.

In den Figuren 2a und 2b ist im Detail ersichtlich, wie der Sensorkopf 14a entlang einer der Vertiefungen 32 bewegt wird. Wie ersichtlich, tritt der Sensorkopf 14a hierfür in die Vertiefung 32 ein. Zur Validierung auch der Achsposition bzw. Achslinearität entlang der Y-Achse kann eine weitere derartige Vermessung der Prüfplatte vorgesehen sein, wobei die Prüfplatte dann beispielsweise entlang der Y-Z-Ebene ausgerichtet und durch ein entsprechend angeordnetes Sensorelement abgefahren werden kann. Auch kann eine Validierung entlang der Y-Achse grundsätzlich dadurch erfolgen, dass bei der in Figur 1 ersichtlichen Anordnung der Prüfplatte 30 in der X-Z-Ebene der Sensorkopf entlang der Y-Achse in eine oder mehrere der Vertiefungen 32 bis 36 hinein und wieder hinaus verfahren wird.

Die erfindungsgemäße Vorrichtung kann an der für das Wasserphantom vorgesehenen Stelle auf einer Patientenliege innerhalb des Magnetfelds des MRT angeordnet und das erfindungsgemäße Verfahren dort durchgeführt werden. Die taktile Erfassung der Prüfplatte 30 durch das taktile Sensorelement 14 wird durch das externe Magnetfeld nicht beeinträchtigt, ganz im Gegensatz zu bisher bekannten Vermessungsverfahren, bei denen ein außerhalb des Wasserphantoms angeordneter Laser auf den Verstellmechanismus gerichtet ist. Die Komponenten der erfindungsgemäßen Vorrichtung, insbesondere der Sensorkopf 14a und die Prüfplatte 30, können in einfacher Weise durch entsprechende Materialwahl MR-tauglich ausgelegt sein. Die Anordnung des Sensorelements innerhalb des Wasserphantoms ist zudem platzsparend und ermöglicht insbesondere die Durchführung des erfindungsgemäßen Verfahrens in der tatsächlichen, späteren Verwendungsposition des Wasserphantoms, nämlich auf der Patientenliege innerhalb des MRT.

Mit der Vorrichtung gemäß Figuren 1 und 2 kann somit in besonders einfacher und zuverlässiger Weise unter realen Bedingungen die Achsposition bzw. Achslinearität des Strahlendetektors entlang aller drei Raumachsen validiert werden. Dasselbe gilt für die nachfolgend erläuterte zweite Ausgestaltung.

Die Figuren 3 bis 6 zeigen eine weitere Ausgestaltung einer erfindungsgemäßen Vorrichtung, die sich von der zuvor erläuterten darin unterscheidet, dass als Normelemente anstatt der Prüfplatte 30 Präzisions-Endmaße 42, 44, 46 vorgesehen sind, die insbesondere aus Keramik bestehen und damit MR-tauglich sein können.

Figur 3 zeigt ein taktiles Sensorelement 14', welches an dem zweiten Schlitten 18 angeordnet ist und somit entlang der Y-Achse beweglich ist. Entlang der Y-Achse erstreckt sich eine Endmaßführung 40, welche in Figur 3 ein Endmaß 42 aufnimmt. In dieser Ansicht ist zudem das zuvor schon angesprochene Aufnahmeelement 15 an der ersten Schiene 16 ersichtlich, das zur Aufnahme des Strahldetektors dient. Bei dieser Ausgestaltung ist jedoch das Sensorelement 14' nicht an diesem Aufnahmeelement angeordnet, wie ersichtlich.

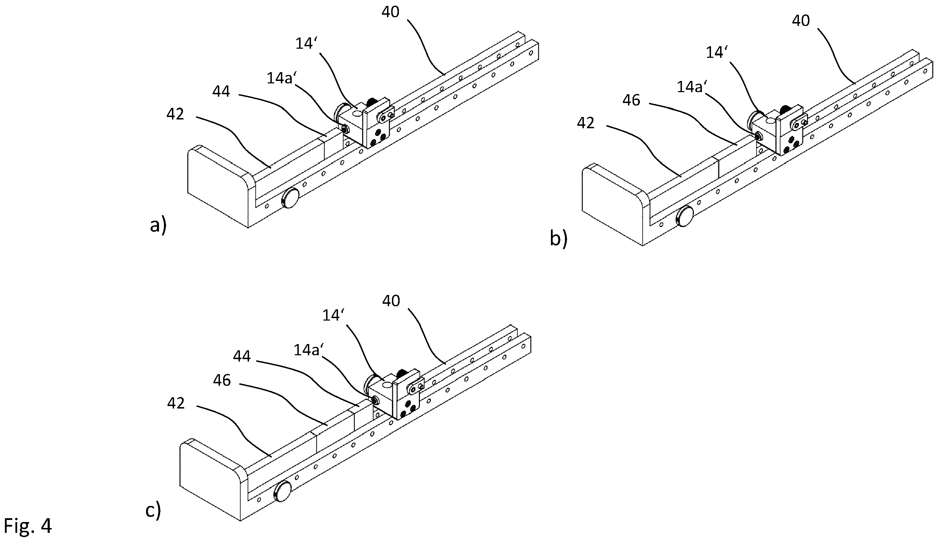

Das Sensorelement 14' weist einen als Kontaktschalter ausgebildeten Sensorkopf 14a' auf, der bei Kontakt mit einem der Endmaße 42, 44, 46 auslöst. Zum taktilen Erfassen der Endmaße wird das Sensorelement 14' entlang der Y-Achse zu dem in Figur 3 ersichtlichen Endmaß 42 hin verstellt, wobei bei Auftreffen des in den Figuren 4 ersichtlichen Kontaktschalters 14a' des Sensorelements 14' der Kontaktschalter 14a' auslöst. Dies wird nachfolgend anhand von Figur 4 erläutert.

In den Figuren 4a-c sind mehrere unterschiedliche Endmaße 42, 44, 46 in der Endmaßführung 40 aufgenommen. Wie ersichtlich, weist ein erstes Endmaß 42 eine erste Länge und ein zweites Endmaß 44 eine zweite Länge auf, wobei die zweite Länge geringer ist als die erste Länge. Zudem weist ein drittes Endmaß 46 eine dritte Länge auf, die zwischen der ersten und der zweiten Länge liegt. Im Rahmen des erfindungsgemäßen Verfahrens werden durch das Sensorelement 14' nun mehrere dieser Endmaße 42, 44, 46 in mehreren Schritten nacheinander taktil erfasst, also durch das Sensorelement 14' derart angefahren, dass der Kontaktschalter 14a' auslöst. So werden zunächst gemäß Figur 4a die Endmaße 42, 44 in die Endmaßführung 40 eingelegt und durch das Sensorelement 14' angefahren. Das Ergebnis ist eine erste Auslöseposition, die beispielsweise durch eine Steuereinheit der Vorrichtung erfasst werden kann. Anschließend wird das Sensorelement 14' wieder von den Endmaßen 42, 44 fort verfahren und es wird das Endmaß 44 ersetzt durch das Endmaß 46, wie Figur 4b zu entnehmen. Im Anschluss wird das Sensorelement 14' an das Endmaß 46 angefahren, sodass wiederum der Kontaktschalter 14a' auslöst und es wird erneut die Auslöseposition vermerkt. Schließlich wird das Sensorelement 14' wieder von dem Endmaß 46 fortgefahren und es wird zusätzlich das Endmaß 44 eingesetzt, wie Figur 4c zu entnehmen. Wiederum wird das Sensorelement 14' zu den Endmaßen hingefahren, sodass der Kontaktschalter 14a' in Kontakt mit dem Endmaß 44 gelangt. Wiederum wird die Auslöseposition vermerkt. Dieses Vorgehen kann für weitere Endmaßlängen wiederholt werden. Die Endmaße können natürlich auch in einer anderen Reihenfolge angelegt und angefahren werden. Wesentlich ist, dass die Endmaße insgesamt in jedem Verfahrschritt eine unterschiedliche Länge bilden.

In einer konkreten Ausführung kann beispielsweise zunächst das Sensorelement 14' in 0,1 mm-Schritten zu den Endmaßen hin verfahren werden, bis der Kontaktschalter 14a' auslöst. Anschließend wird die Auslöseposition notiert. Anschließend wird das Sensorelement 14' um 0,5 mm von den Endmaßen weggefahren. In einem nächsten Schritt kann die das Wasserphantom 12 und damit die gesamte Vorrichtung tragende Patientenliege in das Magnetfeld des MRT verfahren werden. Im Anschluss wird erneut das Sensorelement 14' schrittweise in 0,1 mm-Schritten an dieselben Endmaße herangefahren, bis der Kontaktschalter auslöst. Erneut wird die Auslöseposition notiert. Anschließend wird das Sensorelement 14' um 10 mm von den Endmaßen fortgefahren, dann wiederum um 9,5 mm zu den Endmaßen hin verfahren. Anschließend wird das Sensorelement 14' wiederum in 0,1 mm-Schritten an die Endmaße herangefahren, bis der Kontaktschalter auslöst. Wiederum wird die Auslöseposition notiert. In einem weiteren Schritt wird das Sensorelement 14' um 12 mm von den Endmaßen weggefahren und die Patientenliege anschließend aus dem Magnetfeld verfahren. Anschließend wird das Endmaß um 10 mm verlängert, es kann also ein entsprechendes Endmaß angelegt bzw. das vorherige Endmaß durch ein Endmaß entsprechender Länge ersetzt werden. Im Anschluss wird erneut das Sensorelement 14' in Richtung des Endmaßes verfahren. Diese Schritte werden anschließend wiederholt für drei bis vier unterschiedliche Endmaßlängen.

Auf diese Art und Weise werden im vorliegenden Ausführungsbeispiel mehrere Positionen entlang der Y-Achse validiert, woraus wiederum die Achslinearität validiert werden kann. Eine Durchführung innerhalb sowie außerhalb des Magnetfelds kann zu besonders aussagekräftigen Ergebnissen führen, indem die Messungen innerhalb des Magnetfelds mit denen außerhalb des Magnetfelds verglichen werden. Auch kann für eine wiederholende Qualitätssicherung zunächst nur eine Messung innerhalb des MR und evtl. bei einer Abweichung zur Eingrenzung der Fehler eine Messung auch außerhalb des MR durchgeführt werden. Die Durchführung innerhalb und außerhalb des Magnetfelds kann zudem die Unabhängigkeit des erfindungsgemäßen Verfahrens von dem Magnetfeld bestätigen, wenn gefordert.

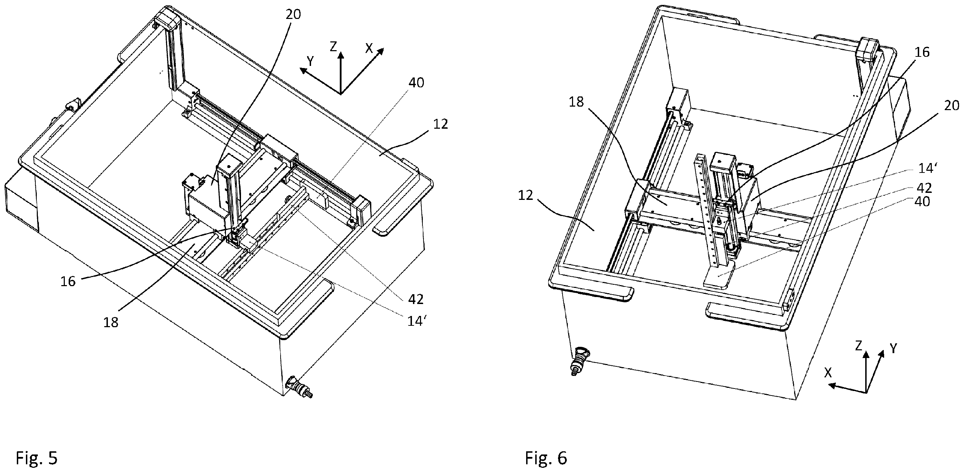

Zur Validierung der Achsposition bzw. Achslinearität auch entlang der X-Achse und der Z-Achse kann die Endmaßführung 40 mit ihren Endmaßen entsprechend entlang den jeweiligen Achsen angeordnet werden, wie Figuren 5 und 6 zu entnehmen. So ist in Figur 5 die Endmaßführung 40 entlang der X-Achse verlaufend angeordnet.

Das Sensorelement 14' ist hierfür an dem Schlitten 16 an dem Aufnahmeelement 15 angeordnet und derart ausgerichtet, dass der Kontaktschalter bei einer Bewegung entlang der X-Achse das Endmaß 42 kontaktiert.

In Figur 6 verläuft die Endmaßführung 40 entlang der Z-Achse. Entsprechend ist das hier ebenfalls an dem Schlitten 16 an dem Aufnahmeelement 15 angeordnete Sensorelement 14' mit seinem Kontaktschalter derart ausgerichtet, dass bei einer Bewegung entlang der Z-Achse der Kontaktschalter das Endmaß 42 kontaktiert. In vergleichbarer Weise - wie zuvor erläutert - kann somit auch entlang der X- und Z-Achse die Achsposition bzw. Achslinearität validiert werden.

In einfacher Weise können die Bauteile der erfindungsgemäßen Vorrichtung MR-tauglich ausgelegt sein. So können mechanische Bauteile aus Keramik bzw. Aluminium bestehen, der Sensorkopf aus nicht ferromagnetischem Metall bzw. als einfacher elektrischer Kontaktschalter. Somit kann die gesamte Vorrichtung, wie bereits angesprochen, innerhalb des Magnetfelds verwendet werden. Auch kann bei mit Wasser gefülltem Phantom das erfindungsgemäße Verfahren durchgeführt werden.

## Patentansprüche

1. Verfahren zur Validierung der Achslinearität und/oder der Positioniergenauigkeit eines Verstellmechanismus für einen Strahlendetektor zum Detektieren von von einem Bestrahlungsgerät ausgesendeter hochenergetischer Strahlung, mit den Schritten: Verstellen eines innerhalb eines Behälters (12) zur Aufnahme einer Flüssigkeit angeordneten, taktilen Sensorelements (14, 14') und/oder eines innerhalb des Behälters (12) angeordneten Normelements (30, 42, 44, 46) über den Verstellmechanismus (16, 18, 20) entlang zumindest einer Raumachse (X, Y, Z) zum taktilen Erfassen des Normelements (30, 42, 44, 46) mittels des taktilen Sensorelements (14, 14').

2. Verfahren nach Anspruch 1, **gekennzeichnet durch** Verstellen des Sensorelements (14, 14') und/oder des Normelements (30, 42, 44, 46) entlang zweier oder dreier zueinander orthogonaler Raumachsen (X, Y, Z) zum taktilen Erfassen des Normelements (30, 42, 44, 46). Auch über Winkel denkbar

3. Verfahren nach Anspruch 1 oder 2, **dadurch gekennzeichnet, dass** Normelement als Prüfplatte (30) mit Vertiefungen (32, 34, 36) und/oder Erhebungen ausgebildet ist, wobei das Sensorelement (14) zum taktilen Erfassen der Prüfplatte (30) entlang der Vertiefungen (32, 34, 36) und/oder Erhebungen gefahren wird und/oder wobei die Prüfplatte (30) mit ihren Vertiefungen (32, 34, 36) und/oder Erhebungen entlang des Sensorelements (14) gefahren wird.

4. Verfahren nach einem der vorhergehenden Ansprüche, **dadurch gekennzeichnet, dass** das Normelement mindestens ein Endmaß (42, 44, 46) umfasst, wobei das Sensorelement (14') zum taktilen Erfassen des Endmaßes (42, 44, 46) in Kontakt mit dem Endmaß (42, 44, 46) gefahren wird und/oder wobei das Endmaß (42, 44, 46) zum taktilen Erfassen des Endmaßes (42, 44, 46) durch das Sensorelement (14') in Kontakt mit dem Sensorelement (14') gefahren wird.

5. Verfahren nach Anspruch 4, **dadurch gekennzeichnet, dass** das Normelement mehrere Endmaße (42, 44, 46) umfasst, die in mehreren Schritten nacheinander durch das Sensorelement (14') taktil erfasst werden.

6. Verfahren nach Anspruch 5, **dadurch gekennzeichnet, dass** einige der Schritte außerhalb und einige der Schritte innerhalb des Magnetfelds eines Magnet-Resonanz-Tomographen (MRT) erfolgen.

7. Vorrichtung zur Validierung der Achslinearität und/oder der Positioniergenauigkeit eines Verstellmechanismus für einen Strahlendetektor zum Detektieren von von einem Bestrahlungsgerät ausgesendeter hochenergetischer Strahlung, umfassend einen Behälter (12) zur Aufnahme einer Flüssigkeit, den Verstellmechanismus und ein innerhalb des Behälters (12) angeordnetes, taktiles Sensorelement (14, 14'), wobei das Sensorelement (14, 14') zum taktilen Erfassen eines innerhalb des Behälters (12) angeordneten Normelements (30, 42, 44, 46) ausgebildet ist, wobei das Sensorelement (14, 14') und/oder das Normelement (30, 42, 44, 46) über den Verstellmechanismus (16, 18, 20) entlang zumindest einer Raumachse (X, Y, Z) relativ zueinander verstellbar sind.

8. Vorrichtung nach Anspruch 7, **dadurch gekennzeichnet, dass** das taktile Sensorelement (14, 14') und/oder das Normelement (30, 42, 44, 46) entlang zweier oder dreier zueinander orthogonaler Raumachsen (X, Y, Z) zum taktilen Erfassen des Normelements (30, 42, 44, 46) verstellbar ist.

9. Vorrichtung nach Anspruch 7 oder 8, **dadurch gekennzeichnet, dass** das Normelement (30) als Prüfplatte mit Vertiefungen (32, 34, 36) und/oder Erhebungen ausgebildet ist, wobei das Sensorelement (14) zum taktilen Erfassen der Prüfplatte (30) entlang der Vertiefungen (32, 34, 36) und/oder Erhebungen fahrbar ist und/oder wobei die Prüfplatte (30) mit ihren Vertiefungen (32, 34, 36) und/oder Erhebungen entlang des Sensorelements (14) fahrbar ist.

10. Vorrichtung nach einem der Ansprüche 7 bis 9, **dadurch gekennzeichnet, dass** das Normelement mindestens ein Endmaß (42, 44, 46) umfasst, wobei das Sensorelement (14') zum taktilen Erfassen des Endmaßes (42, 44, 46) in Kontakt mit dem Endmaß (42, 44, 46) fahrbar ist und/oder wobei das Endmaß (42, 44, 46) zum taktilen Erfassen des Endmaßes (42, 44, 46) durch das Sensorelement (14') in Kontakt mit dem Sensorelement (14') fahrbar ist.

11. Vorrichtung nach Anspruch 10, **dadurch gekennzeichnet, dass** das Normelement mehrere Endmaße (42, 44, 46) umfasst, wobei das Sensorelement (14') zum taktilen Erfassen des jeweiligen Endmaßes (42, 44, 46) nacheinander in Kontakt mit dem jeweiligen Endmaß (42, 44, 46) fahrbar ist und/oder wobei die Endmaße (42, 44, 46) nacheinander zum taktilen Erfassen des jeweiligen Endmaßes (42, 44, 46) durch das Sensorelement (14') in Kontakt mit dem Sensorelement (14') fahrbar sind.

12. Vorrichtung nach einem der Ansprüche 7 bis 11, **dadurch gekennzeichnet, dass** das taktile Sensorelement (14) an der für den Strahlendetektor vorgesehenen Stelle an dem Verstellmechanismus (16) angeordnet ist.

13. Vorrichtung nach einem der Ansprüche 7 bis 12, **gekennzeichnet durch** eine Steuereinheit zur Durchführung des Verfahrens nach einem der Ansprüche 1 bis 6.

14. Verwendung eines Wasserphantoms und dessen Verstellmechanismus für einen Strahlendetektor zum Detektieren von durch ein Bestrahlungsgerät ausgesendeter hochenergetischer Strahlung zur Validierung der Achslinearität und/oder Positioniergenauigkeit des Verstellmechanismus.
